# EUROPEAN PATENT APPLICATION

(11) **EP 4 778 562 A1**
(43) Date of publication of application: **22.07.2026**
(21) Application number: 25152193.6
(22) Date of filing: 16.01.2025
(51) Int. Cl.: A61M 39/10, A61M 39/26

(54) **COMBINED JOINT**

(71) Applicant: Shenzhen Antmed Co., Ltd., Shenzhen, Guangdong 518122 (CN)
(72) Inventor: WANG, Wuxing, Shenzhen, 518122 (CN)
(74) Representative: Monteiro Alves, Inês

(57) **Abstract**

A combined joint includes a sterile infusion joint (1), a connecting joint (2) and a sleeve (3). The sterile infusion joint (1) includes a first shell (11) and a liquid inlet end (12) connected to the first shell (11). A sealing plug bracket (112) and an elastic sealing plug (113) are provided in the first shell (11). The elastic sealing plug (113) is provided in the sealing plug bracket (112), and the elastic sealing plug (113) is provided with a slit (1131). The sleeve (3) is provided on an outside of the sterile infusion joint (1) and is detachably connected to the sterile infusion joint (1). The connecting joint (2) is rotatably connected to the sleeve (3), and the connecting joint (2) includes a connecting pipe (21).

## Description

### TECHNICAL FIELD

The present application relates to the technical field of medical devices, and in particular to a combined joint.

### BACKGROUND

In clinical medicine, according to actual treatment needs, sometimes only a small amount or trace amount of liquid medicine needs to be injected into the patient's body at a time, and a non-disposable infusion line is required at this time. The non-disposable infusion line includes a drug delivery device, a drug delivery tube, a sterile infusion j oint, a connecting j oint, an infusion tube, and an intravenous indwelling needle connected in sequence. When in use, the drug delivery device, the drug delivery tube, and the sterile infusion joint can be reused within a certain period of time, and the connecting joint, the infusion tube, and the intravenous indwelling needle are replaced with the change of patients.

The sterile infusion joint in the related art includes a connector shell and a silicone sealing plug. The silicone sealing plug is provided inside the connector shell and is provided with a slit running through the inside thereof. Under normal conditions of the silicone sealing plug, the slit does not allow liquid to pass through. However, when the silicone sealing plug is deformed, the slit will transform into a liquid hole. When in use, the connector shell of the sterile infusion joint is connected to the connecting joint, and the connecting joint squeezes the silicone sealing plug, and the slit is transformed into a liquid hole to achieve liquid flow. When the preset infusion volume is completed, the infusion needs to be interrupted, the connecting joint is taken out from the connector shell, the sterile infusion joint and the connecting j oint are disconnected, the silicone sealing plug is reset, and the slit is restored to its original state to achieve interrupted infusion. Afterwards, the end face of the silicone sealing plug and the connector shell can be disinfected and wait for the next use.

However, when the infusion is interrupted, the sterile infusion joint is disconnected from the connecting j oint, and the end faces of the connector shell and the silicone sealing plug are exposed to the air. Although disinfection is performed before each use, there is still a risk of contamination of the silicone sealing plug and the end face of the connector shell by air after the disinfection operation.

### SUMMARY

The main purpose of the present application is to provide a combined joint, aiming to solve the technical problem in the related art that an end surface of the sterile infusion joint is easily contaminated. When the infusion needs to be interrupted, after the sterile infusion joint is disconnected from the connecting joint, the end face of the sterile infusion joint is exposed to the air and is easily contaminated.

To achieve the above purpose, the present application provides a combined joint, including: a sterile infusion joint, a connecting joint and a sleeve. The sterile infusion joint includes a first shell and a liquid inlet end connected to the first shell. A sealing plug bracket and an elastic sealing plug are provided in the first shell. The elastic sealing plug is provided in the sealing plug bracket. A slit is provided at an end of the elastic sealing plug, and a first liquid channel is provided inside the elastic sealing plug. When the elastic sealing plug is deformed, the slit is transformed into a first liquid hole. The sleeve is provided on an outside of the sterile infusion joint and is detachably connected to the sterile infusion joint. The connecting joint is rotatably connected to the sleeve, and the connecting joint includes a connecting pipe. The connecting pipe is configured to extend to an inside of the sleeve, and the sleeve is provided on an outside of the first shell. When the sleeve is close to the liquid inlet end, the connecting pipe is extended into an inside of the first shell. And when the sleeve is away from the liquid inlet end, the connecting pipe is kept away from the first shell.

In an embodiment, the sleeve includes a cylinder and an assembly plate; the assembly plate is provided at one end of the cylinder, and the connecting joint is provided at the assembly plate and is rotatably connected to the cylinder; and the cylinder is provided on the outside of the first shell.

In an embodiment, the first shell is connected to the cylinder by threads.

In an embodiment, the connecting joint further includes a joint end, an abutment plate and a protrusion; the joint end is communicated with the connecting pipe; the protrusion is provided on a side of the abutment plate away from the joint end; a groove is formed between the protrusion and the abutment plate, and a side of the assembly plate away from the cylinder is movably abutted against the abutment plate to enable a relative rotation of the sleeve and the connecting joint.

In an embodiment, a first mounting ring is provided at an end of the first shell facing the liquid inlet end; a raised platform is provided on an outer wall of the first mounting ring along a circumferential direction of the first mounting ring; a second mounting ring is provided at an end of the liquid inlet end facing the first shell; an inner diameter of the second mounting ring is adapted to an outer diameter of the first mounting ring, and the second mounting ring is provided on an outer side of the first mounting ring and abutted against the raised platform.

In an embodiment, the sealing plug bracket includes a connecting part and a connecting shell; the connecting part is provided at an end of the connecting shell and connected to an inner wall of the first shell; a receiving cavity is provided in the connecting shell; the elastic sealing plug is tightly filled in the receiving cavity, and a plurality of connecting ribs are provided between an outer wall of the connecting shell and the inner wall of the first shell.

In an embodiment, the elastic sealing plug is a silicone sealing plug.

In an embodiment, the sterile infusion joint further includes a support plate, a first support ring and a second support ring; the first support ring is connected to one side of the support plate, and the second support ring is connected to the other side of the support plate.

In an embodiment, a second liquid channel is provided at the liquid inlet end; the sterile infusion joint further includes a backflow blocking member, and the backflow blocking member includes a block and a silicone seal component; the block is connected to an inner side wall of the second liquid channel; a second liquid hole is provided between the inner side wall of the second liquid channel and the block; the silicone seal component is provided on an inner side of the second support ring and is abutted against the second support ring; the silicone seal component is provided with a third liquid hole, and a position of the third liquid hole is adapted to the block.

In an embodiment, the joint end is a straight joint, a male joint, a female joint or a three-way j oint, and the liquid inlet end is a straight j oint, a male j oint, a female joint or a three-way joint.

The present application discloses a combined j oint, including a sterile infusion joint, a connecting joint, and a sleeve. The sterile infusion joint includes a first shell and a liquid inlet end connected to the first shell. The sleeve is provided at the outer side of the sterile infusion joint and is detachably connected to the sterile infusion joint, and the sleeve is rotatably connected to the connecting j oint. In the present application, by setting a detachable connection between the sleeve and the sterile infusion joint, the sleeve is screwed to the outside of the sterile infusion joint, and the sleeve is rotatably connected to the connecting joint, which has the characteristics of simple operation and novel design. When in use, the relative position of the sleeve and the sterile infusion joint is adjusted so that the sleeve is close to the liquid inlet end, and the connecting joint is driven to extend into the first shell. Or, the relative position of the sleeve and the sterile infusion joint is adjusted so that the sleeve is away from the liquid inlet end, so as to drive the connecting joint away from the first shell. During use, the first shell, the elastic sealing plug, and the sleeve are always inside the sleeve, which effectively solves the technical problem in the related technology that when the infusion needs to be interrupted, after the sterile infusion joint is disconnected from the connecting joint, the end face of the sterile infusion joint is exposed to the air, which is easy to contaminate the sterile infusion joint.

### BRIEF DESCRIPTION OF THE DRAWINGS

In order to more clearly illustrate the embodiments of the present application or the technical solutions in the related art, the drawings required for use in the embodiments or the description of the related art will be briefly introduced below. Obviously, the drawings described below are only some embodiments of the present application. For those skilled in the art, other drawings can be obtained based on the structures shown in these drawings without creative work.
FIG. 1 is an overall structural view of a combined joint according to an embodiment of the present application.
FIG. 2 is a cross-sectional view of the combined joint in a first position according to an embodiment of the present application.
FIG. 3 is a cross-sectional view of the combined joint in a second position according to an embodiment of the present application.
FIG. 4 is an enlarged view of part A in FIG. 3.
FIG. 5 is an enlarged view of part B in FIG. 3.

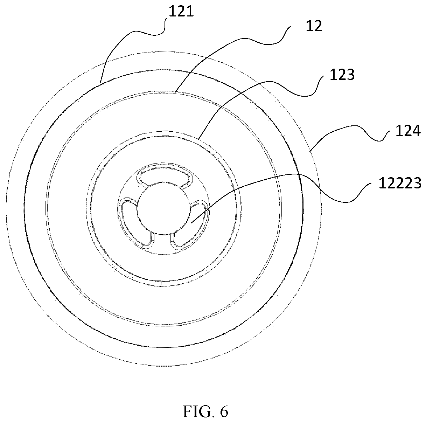

FIG. 6 is a schematic cross-sectional view of a sterile infusion joint and a connecting joint when they are in a connected state according to an embodiment of the present application.

Description of reference signs: 1. sterile infusion joint; 11. first shell; 110. first cavity; 111. first mounting ring; 112. sealing plug bracket; 1121. connecting part; 1122. connecting shell; 1123. connecting rib; 113. elastic sealing plug; 1131. slit; 1132. first liquid channel; 1133. abutment section; 1134. filling section; 114. first thread; 115. raised platform; 12. liquid inlet end; 121. first bracket; 1211. support plate; 1212. first support ring; 1213. second support ring; 1214, first liquid hole; 122, backflow blocking member; 1221, block; 1222, silicone seal component; 12221, sealing cover; 12222, sealing ring; 12223, second liquid hole; 12224, third liquid hole; 123, second liquid channel; 124, second mounting ring; 2, connecting joint; 21, connecting pipe; 22, joint end; 23, abutment plate; 24, protrusion; 241, groove; 3, sleeve; 31, assembly plate; 311, assembly hole; 32, cylinder; 321, second thread; 33, anti-slip rib; 4, wing.

The realization of the purpose, functional features and advantages of the present application will be further explained in conjunction with embodiments and with reference to the accompanying drawings.

### DETAILED DESCRIPTION OF THE EMBODIMENTS

The following will be combined with the drawings in the embodiments of the present application to clearly and completely describe the technical solutions in the embodiments of the present application. Obviously, the described embodiments are part of the embodiments of the present application, not all of the embodiments. Based on the embodiments of the present application, all other embodiments obtained by those skilled in the art without creative work belong to the scope of protection of the present application.

It should be understood that when used in this specification and the appended claims, the terms "include" and "comprise" indicate the existence of the described features, wholes, steps, operations, elements and/or components, but do not exclude the existence or addition of one or more other features, wholes, steps, operations, elements, components and/or their collections.

It should also be understood that the terms used in the present specification are only for the purpose of describing specific embodiments and are not intended to limit the present application. As used in the present specification and the appended claims, the singular forms "one", "an" and "the" are intended to include the plural forms unless the context clearly indicates otherwise.

It should also be further understood that the terms "and, or" used in the present specification and the appended claims refer to any combination of one or more of the associated listed items and all possible combinations, and include these combinations.

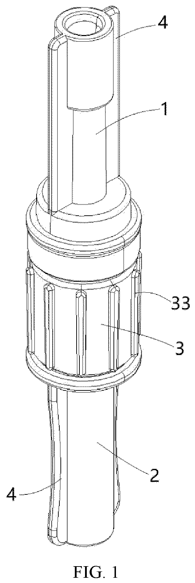

The present application provides a combined j oint, as shown in FIG. 1, the combined joint includes: a sterile infusion joint 1, a connecting joint 2 and a sleeve 3. The sterile infusion joint 1 includes a first shell 11 and a liquid inlet end 12 connected to the first shell 11. The sleeve 3 is provided on the outside of the sterile infusion joint 1 and is detachably connected to the sterile infusion joint 1. After the sleeve 3 is communicated with the sterile infusion joint 1, the position of the sleeve 3 relative to the sterile infusion joint 1 can be changed on the same plane.

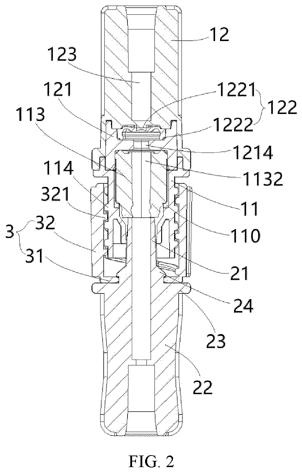

As shown in FIG. 2, the first shell 11 and the liquid inlet end 12 are connected in cooperation. The first shell 11 and the liquid inlet end 12 are enclosed to form an installation cavity. The first bracket 121 and the backflow blocking member 122 are provided at the installation cavity. A first cavity 110 is provided at the first shell 11. A sealing plug bracket 112 and an elastic sealing plug 113 are provided inside the first cavity 110. The elastic sealing plug 113 is assembled in the sealing plug bracket 112. The elastic sealing plug 113 is provided at one side of the first bracket 121, and the backflow blocking member 122 is provided at the other side of the first bracket 121. A slit 1131 is provided at the end of the elastic sealing plug 113. When the elastic sealing plug 113 is squeezed and deformed, the slit 1131 is transformed into a first liquid hole 1214. When the elastic sealing plug 113 is not squeezed, the slit 1131 is in a closed state, and the flow of the liquid medicine is not allowed. The elastic sealing plug 113 is provided with a first liquid channel 1132 inside, and the liquid inlet end 12 is provided with a second liquid channel 123. The backflow blocking member 122 can be an elastic member, and the one-way conduction of the liquid can be realized through the elastic deformation of the elastic member. In an embodiment, the backflow blocking member 122 keeps the first liquid hole 1214 closed in a normal state to block the liquid flow between the second liquid channel 123 and the first liquid channel 1132. When the backflow blocking member 122 is squeezed, the first liquid hole 1214 is opened, so that the second liquid channel 123 is communicated with the first liquid channel 1132.

It can be understood that, in an embodiment, the backflow blocking member 122 can also be a one-way control valve core, such as a butterfly valve core and a ball valve core.

The elastic sealing plug 113 is provided inside the first cavity 110, which is not easy for the user to touch during use, so the combined joint of this embodiment has the effect of preventing contamination.

After the sleeve 3 is communicated with the sterile infusion joint 1, the position of the sleeve 3 relative to the sterile infusion joint 1 can be changed on the same plane. For example, the position of the sleeve 3 relative to the sterile infusion joint 1 on the same plane can be multiple. Because the position of the sleeve 3 relative to the sterile infusion joint 1 on the same plane can be changed, after the sleeve 3 is communicated with the sterile infusion joint 1, the sleeve 3 can be close to the liquid inlet end 12 or away from the liquid inlet end 12.

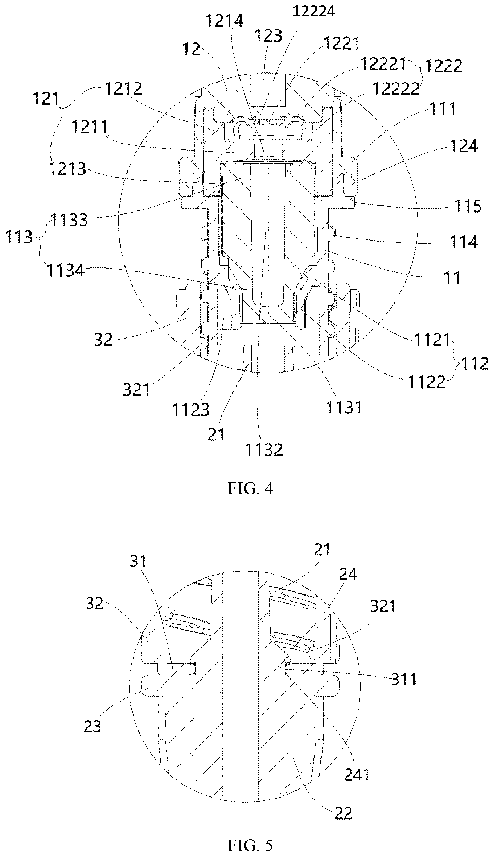

As shown in FIG. 4, the end of the first shell 11 facing the liquid inlet end 12 is provided with a first mounting ring 111, and the outer wall of the first mounting ring 111 is provided with a raised platform 115 along the circumferential direction of the first mounting ring 111. The end of the liquid inlet end 12 facing the first shell 11 is provided with a second mounting ring 124, and the inner diameter of the second mounting ring 124 is adapted to the outer diameter of the first mounting ring 111. The second mounting ring 124 is provided on the outside of the first mounting ring 111, and the end of the second mounting ring 124 away from the liquid inlet end 12 is abutted against the raised platform 115. In this embodiment, the outer wall of the first mounting ring 111 is tightly fitted with the inner wall of the second mounting ring 124. For example, the joint between the outer wall of the first mounting ring 111 and the inner wall of the second mounting ring 124 can be tightly connected by ultrasonic welding.

The outer wall of the liquid inlet end 12 is also provided with a wing 4, which is convenient for the user to hold the wing 4 for assembly operation.

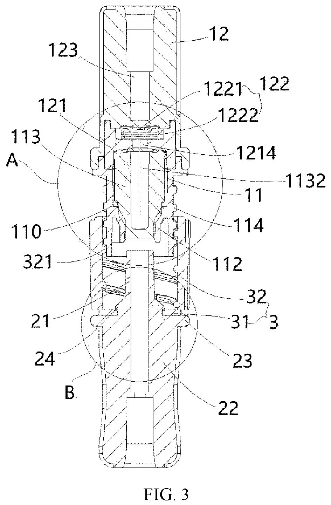

The connecting joint 2 is rotatably connected to the sleeve 3, and the connecting joint 2 and the sleeve 3 can rotate relative to each other. The connecting joint 2 includes a connecting pipe 21, which extends to the inside of the sleeve 3. When the sleeve 3 is assembled on the outside of the sterile infusion joint 1, the position of the connecting pipe 21 corresponds to the position of the elastic sealing plug 113. During assembly, by adjusting the relative position of the sleeve 3 and the sterile infusion joint 1, the sleeve 3 can be close to the liquid inlet end 12, so that the connecting pipe 21 can be inserted into the inside of the first shell 11. When the connecting pipe 21 is inserted into the inside of the first shell 11, it will squeeze the elastic sealing plug 113, so that the slit 1131 is transformed into the first liquid hole 1214. When the slit 1131 is transformed into the first liquid hole 1214, the sterile infusion joint 1 is communicated with the connecting joint 2, and the liquid medicine can flow from the sterile infusion joint 1 to the connecting joint 2. In this case, as shown in FIG. 2, the sleeve 3 is in the first position relative to the sterile infusion joint 1. During assembly, by adjusting the relative position of the sleeve 3 and the sterile infusion joint 1, the sleeve 3 can also be moved away from the liquid inlet end 12, and the connecting pipe 21 can be moved away from the first shell 11. In this case, the elastic sealing plug 113 returns to its original state due to the loss of force, so that the slit is closed, the sterile infusion joint 1 and the connecting joint 2 are disconnected, and the liquid flow is blocked. In this case, as shown in FIG. 3, the sleeve 3 is in the second position relative to the sterile infusion joint 1.

The outer wall of the sleeve 3 is provided with an anti-slip rib 33 configured to anti-slip, as shown in FIG. 1.

The sleeve 3 and the sterile infusion joint 1 can be detachably connected in a manner that the first shell 11 and the sleeve 3 are connected by threaded connection. In an embodiment, the outer wall of the first shell 11 is provided with a first thread 114, and the inner wall of the sleeve 3 is provided with a second thread 321 adapted to the first thread 114, so as to realize the threaded connection between the sleeve 3 and the first shell 11. During assembly, the user can screw the sleeve 3 to the outer wall of the first shell 11, so that the sleeve 3 is close to the liquid inlet end 12, and the connecting pipe 21 is close to the first shell 11. Alternatively, the user can screw the sleeve 3 to the outer wall of the first shell 11, so that the sleeve 3 is away from the liquid inlet end 12, and the connecting pipe 21 is kept away from the first shell 11.

The sleeve 3 includes a cylinder 32 and an assembly plate 31. The cylinder 32 is cylindrical and is provided with a cavity inside. The assembly plate 31 is covered at one end of the cylinder 32. An assembly hole 311 is provided at the center of the assembly plate 31. The connecting joint 2 is assembled at the assembly hole 311. The cylinder 32 is coaxially provided with the connecting joint 2 and the connecting joint 2 is rotatably connected to the cylinder 32. The second thread 321 is provided on the inner wall of the cylinder 32. The cylinder 32 is assembled on the outer side of the first shell 11.

The connecting joint 2 also includes a joint end 22, an abutment plate 23, and a protrusion 24. As shown in FIG. 5, the joint end 22 is communicated with the connecting pipe 21. The joint end 22 is provided at one side of the abutment plate 23, and the connecting pipe 21 is provided at the other side of the abutment plate 23. The abutment plate 23 is provided to protrude along the circumferential direction of the outer wall of the joint end 22 and is provided perpendicular to the joint end 22. The protrusion 24 is provided on the side of the abutment plate 23 away from the joint end 22. The protrusion 24 is provided to protrude along the circumferential direction of the outer wall of the connecting pipe 21. A groove 241 is formed between the protrusion 24 and the abutment plate 23, and the assembly hole 311 is assembled at the groove 241. The size of the groove 241 is consistent with the size of the assembly hole 311, and there is a rotation gap between the groove 241 and the assembly hole 311, so that the sleeve 3 can be flexibly rotated relative to the groove 241. The side of the assembly plate 31 away from the cylinder 32 is movably abutted against the abutment plate 23, and the side of the assembly plate 31 close to the cylinder 32 is movably abutted against the protrusion 24. There is an assembly gap at the abutment. The cylinder 32 is screwed to the outer wall of the first shell 11, and the sleeve 3 gradually approaches the liquid inlet end 12, driving the connecting joint 2 to move toward the first shell 11. At the same time, the connecting joint 2 and the cylinder 32 rotate relative to each other, and the infusion tube communicated with the connecting joint 2 can also be prevented from being knotted.

The outer wall of the joint end 22 is also provided with a wing 4, and the user can hold the wing 4 for assembly operation.

As shown in FIG. 4, the sealing plug bracket 112 includes a connecting part 1121 and a connecting shell 1122. The connecting part 1121 is provided at the end of the connecting shell 1122 and connected to the inner wall of the first shell 11. The connecting shell 1122 is provided with a receiving cavity, and the elastic sealing plug 113 is tightly filled in the receiving cavity. A plurality of connecting ribs 1123 are provided between the outer wall of the connecting shell 1122 and the inner wall of the first shell 11. When in use, the sleeve 3 is adjusted to be close to the liquid inlet end 12, so that the connecting pipe 21 is extended into the interior of the connecting shell 1122. Alternatively, the sleeve 3 is adjusted to be away from the liquid inlet end 12, so that the connecting pipe 21 is away from the connecting shell 1122. When a force is applied to the elastic sealing plug 113, the elastic sealing plug 113 and the connecting shell 1122 are subjected to external force, and the connecting rib 1123 can prevent the connecting shell 1122 from being deformed, thereby extending the service life of the connecting shell 1122.

The elastic sealing plug 113 includes a filling section 1134 and an abutment section 1133. The shape of the filling section 1134 matches the shape of the connecting shell 1122. The filling section 1134 is tightly filled in the connecting shell 1122. The abutment section 1133 is abutted against the first bracket 121. The first bracket 121 is configured to limit the elastic sealing plug 113. The slit 1131 runs through the filling section 1134 to open or close the first liquid channel 1132. In this embodiment, the elastic sealing plug 113 is a silicone sealing plug. The silicone sealing plug 113 has high elasticity, and will deform when compressed, so that the first liquid channel 1132 can be quickly opened, and the first liquid channel 1132 can be reset and closed after pressure relief.

The first bracket 121 includes a support plate 1211, a first support ring 1212 and a second support ring 1213. The first support ring 1212 is connected to one side of the support plate 1211, and the second support ring 1213 is connected to the other side of the support plate 1211. The cross section of the first bracket 121 is "H" shaped. The first liquid hole 1214 is provided at the center of the support plate 1211 and the position of the first liquid hole 1214 corresponds to the position of the first liquid channel 1132. The abutment section 1133 of the elastic sealing plug 113 is abutted against the inner side of the first support ring 1212. One end of the abutment section 1133 is abutted against the inner side wall of the first support ring 1212, and the other end of the abutment section 1133 is abutted against the support plate 1211. The outer side wall of the end of the abutment section 1133 is protruded with a flange toward the first support ring 1212, and the flange is abutted against the inner side wall of the first support ring 1212. The end of the abutment section 1133 is protruded with a first convex ring toward the support plate 1211. The first convex ring is abutted against the support plate 1211, so that the abutment section 1133 is tightly engaged. The backflow blocking member 122 is provided on the inner side of the second support ring 1213 and is abutted against the support plate 1211.

In an embodiment, the backflow blocking member 122 includes a block 1221 and a silicone seal component 1222. The block 1221 is connected to the inner side wall of the second liquid channel 123. A second liquid hole 12223 is provided between the block 1221 and the inner side wall of the second liquid channel 123. The silicone seal component 1222 is provided on the inner side of the second support ring 1213 and is abutted against the second support ring 1213. The silicone seal component 1222 includes a sealing ring 12222 and a sealing cover 12221. The sealing cover 12221 is covered at one end of the silicone seal component 1222. The sealing cover 12221 is provided with a third liquid hole 12224. The position of the third liquid hole 12224 corresponds to the position of the block 1221. The outer wall of the silicone seal component 1222 is abutted against the inner wall of the second support ring 1213, and the end of the sealing ring 12222 away from the sealing cover 12221 is abutted against the support plate 1211. The liquid flows from the sterile infusion joint 1 to the top of the sealing ring 12222 through the second liquid hole 12223. Under the action of the gravity of the liquid, the liquid presses the sealing ring 12222 downward, and a liquid flow channel is formed between the sealing ring 12222 and the block 1221 to realize the liquid flowing from the second liquid channel 123 to the first liquid channel 1132. In case of liquid backflow, the liquid presses the sealing ring 12222 toward the block 1221, blocking the second liquid hole 12223, and the liquid cannot continue to flow to the liquid inlet end 12, thereby realizing the unidirectional flow of the liquid.

The j oint end 22 is a straight j oint, a male j oint, a female j oint, or a three-way j oint, and the liquid inlet end 12 is a straight joint, a male joint, a female joint, or a three-way joint. The types of the joint end and the liquid inlet end are set according to the usage scenario, which can meet the requirements of applying the combined joint to infusion pipelines containing different types of connecting joints.

The combined joint also includes a first protective cap (not shown in the figure) and a second protective cap (not shown in the figure). When the product is assembled and packaged, the first protective cap is provided at the end of the first shell 11, and the first protective cap and the sterile infusion joint 1 are packaged separately. The first protective cap is configured to protect the sterile infusion joint 1 from contamination during transportation. The connecting joint 2 is assembled at the assembly hole 311 of the sleeve 3, and the second protective cap is provided at the end of the sleeve 3. The second protective cap, the connecting joint, and the sleeve are packaged separately. The second protective cap is configured to protect the connecting joint 2 and the sleeve 3 from contamination during transportation. After the product is transported to the user, the user removes the first protective cap and the second protective cap respectively, screws the sleeve 3 to the outside of the first shell 11, and then starts to use it.

In an embodiment, the joint end 22 is set as a straight joint, and the liquid inlet end 12 is set as a straight j oint, so as to obtain a combined joint as shown in FIG. 1. The combined joint is communicated with a non-disposable infusion pipeline, and the liquid inlet end 12 is communicated with the drug delivery device through the drug delivery tube. The joint end 22 is communicated with the connecting joint, the infusion tube, and the intravenous indwelling needle in sequence. When infusion is required, the sleeve 3 is screwed to the outside of the first shell 11 and close to the liquid inlet end 12, driving the connecting pipe 21 to extend into the interior of the first shell 11. The elastic sealing plug 113 is subjected to the force of the connecting pipe 21 and is squeezed by the connecting pipe 21. In this case, the slit 1131 is transformed into the first liquid hole 1214, connecting the sterile infusion joint 1 and the connecting joint 2, and infusion is performed. When the infusion needs to be interrupted, the sleeve 3 is screwed to move the sleeve 3 away from the liquid inlet end 12, driving the connecting pipe 21 away from the first shell 11, so that the elastic sealing plug 113 loses its force and returns to its original state, the slit 1131 is closed, the sterile infusion joint 1 and the connecting joint 2 are disconnected, and the infusion is interrupted. During use, the first shell 11, the elastic sealing plug 113, and the connecting pipe 21 are provided inside the sleeve 3, which can effectively avoid contamination of the elastic sealing plug 113. If the connecting joint 2 and the sleeve 3 need to be replaced, the sleeve 3 is screwed out from the outside of the first shell 11 and replaced according to the use requirements.

The present application discloses a combined j oint, including a sterile infusion joint, a connecting joint, and a sleeve. The sleeve is provided at the outer side of the sterile infusion joint and is detachably connected to the sterile infusion joint. The sleeve is rotatably connected to the connecting joint, so that the relative position of the sleeve and the sterile infusion joint can be changed, so that the sleeve is close to or away from the liquid inlet end. The combined joint disclosed by the present application has the characteristics of simple operation and novel design. The combined joint is connected in a non-disposable infusion pipeline. When in use, by adjusting the relative position of the sleeve and the sterile infusion joint, the sleeve is close to or away from the liquid inlet end, driving the connecting joint to extend into the first shell or away from the first shell, so as to realize the connection or disconnection of the sterile infusion joint and the connecting joint. During use, the first shell, the elastic sealing plug, and the sleeve are always inside the sleeve, which effectively solves the technical problem in the related art that if the infusion needs to be interrupted, the sterile infusion joint needs to be disconnected from the connecting j oint, which makes the end face of the sterile infusion joint exposed to the air, and easily contaminated. Moreover, this technology uses a backflow blocking member in conjunction with an elastic sealing plug to effectively prevent the backflow of the drug solution and contamination of the drug delivery device when the infusion is interrupted. Furthermore, during use, when the sleeve is screwed to the outside of the sterile infusion joint, the connecting joint is rotated relative to the sleeve, thereby preventing the infusion tube communicated with the connecting joint from becoming tangled, thereby alleviating the discomfort of the patient.

The above contents are only some embodiments of the present application, and do not limit the scope of the present application. All equivalent structural changes made by using the contents of the present application specification and drawings under the inventive concept of the present application, or directly/indirectly applied in other related technical fields are included in the scope of the present application.

## Claims

1. A combined joint, **characterized by** comprising:
a sterile infusion joint (1);
a connecting joint (2); and
a sleeve (3);
wherein the sterile infusion joint (1) comprises a first shell (11) and a liquid inlet end (12) connected to the first shell (11);
a sealing plug bracket (112) and an elastic sealing plug (113) are provided in the first shell (11);
the elastic sealing plug (113) is provided in the sealing plug bracket (112);
a slit (1131) is provided at an end of the elastic sealing plug (113), and a first liquid channel (1132) is provided inside the elastic sealing plug (113);
when the elastic sealing plug (113) is deformed, the slit (1131) is transformed into a first liquid hole (1214);
the sleeve (3) is provided on an outside of the sterile infusion joint (1) and is detachably connected to the sterile infusion joint (1);
the connecting joint (2) is rotatably connected to the sleeve (3), and the connecting joint (2) comprises a connecting pipe (21);
the connecting pipe (21) is configured to extend to an inside of the sleeve (3), and the sleeve (3) is provided on an outside of the first shell (11);
when the sleeve (3) is close to the liquid inlet end (12), the connecting pipe (21) is extended into an inside of the first shell (11); and
when the sleeve (3) is away from the liquid inlet end (12), the connecting pipe (21) is kept away from the first shell (11).

2. The combined joint according to claim 1, wherein the sleeve (3) comprises a cylinder (32) and an assembly plate (31); the assembly plate (31) is provided at one end of the cylinder (32), and the connecting joint (2) is provided at the assembly plate (31) and is rotatably connected to the cylinder (32); and the cylinder (32) is provided on the outside of the first shell (11).

3. The combined joint according to claim 2, wherein the first shell (11) is connected to the cylinder (32) by threads.

4. The combined joint according to claim 3, wherein the connecting joint (2) further comprises a joint end (22), an abutment plate (23) and a protrusion (24); the joint end (22) is communicated with the connecting pipe (21); the protrusion (24) is provided on a side of the abutment plate (23) away from the joint end (22); a groove (241) is formed between the protrusion (24) and the abutment plate (23), and a side of the assembly plate (31) away from the cylinder (32) is movably abutted against the abutment plate (23) to enable a relative rotation of the sleeve (3) and the connecting joint (2).

5. The combined joint according to claim 1, wherein a first mounting ring (111) is provided at an end of the first shell (11) facing the liquid inlet end (12); a raised platform (115) is provided on an outer wall of the first mounting ring (111) along a circumferential direction of the first mounting ring (111); a second mounting ring (124) is provided at an end of the liquid inlet end (12) facing the first shell (11); an inner diameter of the second mounting ring (124) is adapted to an outer diameter of the first mounting ring (111), and the second mounting ring (124) is provided on an outer side of the first mounting ring (111) and abutted against the raised platform (115).

6. The combined joint according to claim 1, wherein the sealing plug bracket (112) comprises a connecting part (1121) and a connecting shell (1122); the connecting part (1121) is provided at an end of the connecting shell (1122) and connected to an inner wall of the first shell (11); a receiving cavity is provided in the connecting shell (1122); the elastic sealing plug (113) is tightly filled in the receiving cavity, and a plurality of connecting ribs (1123) are provided between an outer wall of the connecting shell (1122) and the inner wall of the first shell (11).

7. The combined joint according to claim 6, wherein the elastic sealing plug (113) is a silicone sealing plug.

8. The combined joint according to claim 7, wherein the sterile infusion joint (1) further comprises a support plate (1211), a first support ring (1212) and a second support ring (1213); the first support ring (1212) is connected to one side of the support plate (1211), and the second support ring (1213) is connected to the other side of the support plate (1211).

9. The combined joint according to claim 8, wherein a second liquid channel (123) is provided at the liquid inlet end (12); the sterile infusion joint (1) further comprises a backflow blocking member (122), and the backflow blocking member (122) comprises a block (1221) and a silicone seal component (1222); the block (1221) is connected to an inner side wall of the second liquid channel (123); a second liquid hole (12223) is provided between the inner side wall of the second liquid channel (123) and the block (1221); the silicone seal component (1222) is provided on an inner side of the second support ring (1213) and is abutted against the second support ring (1213); the silicone seal component (1222) is provided with a third liquid hole (12224), and a position of the third liquid hole (12224) is adapted to the block (1221).

10. The combined joint according to claim 4, wherein the joint end (22) is a straight joint, a male j oint, a female joint or a three-way joint, and the liquid inlet end (12) is a straight joint, a male j oint, a female joint or a three-way joint.
